# EUROPEAN PATENT APPLICATION

(11) **EP 1 104 771 A1**
(43) Date of publication of application: **06.06.2001**
(21) Application number: 99935092.9
(22) Date of filing: 06.08.1999
(51) Int. Cl.: C07K 14/47, C07K 16/18, C12N 15/12, C12P 21/02, A61K 38/17

(54) **NOVEL POLYPEPTIDE, cDNA ENCODING THE SAME AND UTILIZATION THEREOF**

(30) Priority: 07.08.1998 JP 22430898
(71) Applicant: ONO PHARMACEUTICAL CO., LTD., Osaka-shi, Osaka 541-8526 (JP)
(72) Inventor: FUKUSHIMA, Daikichi, Mishima-gun, Osaka 618-8585 (JP); SHIBAYAMA, Shiro, Mishima-gun, Osaka 618-8585 (JP); TADA, Hideaki, Mishima-gun, Osaka 618-8585 (JP)
(74) Representative: Henkel, Feiler, Hänzel
(86) International application number: JP9904265
(87) International publication number: WO0008056

(57) **Abstract**

A new human polypeptide, a cDNA encoding the same and a pharmaceutical use of it.

The polypeptides of the present invention possess hematopoiesis regulating activity, tissue generation/regeneration activity, activin/inhibin activity, chemotactic/chemokinetic activity, hemostatic and thrombolytic activity, and receptor/ligand activity, therefore, they are expected to be useful for prevention and/or treatment of various diseases.

## Description

### Technical Field

The present invention relates to a novel polypeptide, a method for producing it, a cDNA encoding it, a vector containing the cDNA, a host cell transformed with the vector, an antibody against the peptide, and a pharmaceutical composition containing the polypeptide or the antibody.

### Technical Background

Until now, when a man skilled in the art intends to obtain a particular polypeptide or a cDNA encoding it, he generally utilizes methods by confirming an aimed biological activity in a tissue or in a cell medium, isolating and purifying the polypeptide and then cloning a gene or methods by "expression-cloning" with the guidance of the said biological activity. However, physiologically active polypeptides in living body have often many kinds of activities. Therefore, it happens increasingly that after cloning a gene, the isolated gene is found to be identical to that encoding a polypeptide already known. In addition, some factors could be generated in only a very slight amount and/or under specific conditions and it makes difficult to isolate and to purify the factor and to confirm its biological activity.

Recent rapid developments in techniques for constructing cDNAs and sequencing techniques have made it possible to quickly sequence a large amount of cDNAs. By utilizing these techniques, a process, which comprises constructing cDNAs library using various cells or tissues, cloning the cDNA at random, identifying the nucleotide sequences thereof, expressing novel polypeptides encoded by them, is now in progress. Although this process is advantageous in that a gene can be cloned and information regarding its nucleotide sequence can be obtained without any biochemical or genetic analysis, the target gene can be discovered thereby only accidentally in many cases.

### Disclosure of the Invention

The present inventors have studied cloning method to isolate genes encoding proliferation and/or differentiation factors functioning in hematopoietic systems and immune systems. Focusing their attention on the fact that most of the secretory proteins such as proliferation and/or differentiation factors (for example, various cytokines) and membrane proteins such as receptors thereof (hereafter these proteins will be referred to generally as secretory proteins and the like) have sequences called signal peptides in the N-termini, the inventors have conducted extensive studies on a process for efficiently and selectively cloning a gene encoding for a signal peptide. Finally, we have successfully developed a screening method for the signal peptides (signal sequence trap (SST)) by using mammalian cells (See EP-0607054). We also developed yeast SST method on the same concept. By the method based on the same conception using yeast, (yeast SST method), genes including sequence encoding signal peptide can be identified more easily and efficiently (See USP No. 5,536,637).

The present inventors et al. have diligently performed certain investigation in order to isolate novel factors (polypeptides) useful for treatment, diagnosis and/or study, particularly, secretory proteins containing secretory signal and membrane protein. From the result, the present inventors achieved to find novel secretory proteins and membrane proteins produced from cell lines and tissue, for example, human placenta, human adult brain tissue, cell lines derived from human brain tissue, human bone, cell line derived from human bone marrow, and endothelial cell line of vein derived from human umbilical cord (HUV-EC-C) and cDNAs encoding them, and then completed the present invention.

A cDNA nucleotide sequence provided by the present invention was identified as clone 0AH047. The said clone was isolated from cDNA library synthesized from endothelial cell line of vein derived from human umbilical cord (HUV-EC-C) based on the information obtained by using the above yeast SST method. Clone 0AH047 was full-length cDNA containing nucleotide sequence encoding membrane protein (represented as 0AH047 protein).

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence and the nucleotide sequence of 0AH047 of the present invention. It was also indicated from the hydrophobisity analysis that the polypeptide of the present invention had no transmembrane region. Taken altogether, it was proved that polypeptide 0AH047 of the present invention was new secretary protein.

That is to say, the invention relates to:
(1) a polypeptide comprising an amino acid sequence shown in SEQ ID NO. 1,
(2) a cDNA encoding the polypeptide described in above (1),
(3) a cDNA comprising a nucleotide sequence shown in SEQ ID NO. 2,
(4) a cDNA comprising a nucleotide sequence shown in SEQ ID NO. 3.

### Detailed Description of the present invention

The present invention relates to a polypeptide comprising amino acid sequence shown in SEQ ID NO. 1 in substantially purified form, a homologue thereof, a fragment of the sequence and a homologue of the fragment.

Further, the present invention relates to cDNAs encoding the above peptides. More particularly the invention is provided cDNAs comprising nucleotide sequence shown in SEQ ID NO. 2 and cDNA containing a fragment which is selectively hybridizing to the cDNA comprising nucleotide sequences shown in SEQ ID NOS. 2 or 3. A said cDNA capable for hybridizing to the cDNA includes the contemporary sequence of the above sequence.

A polypeptide comprising amino acid sequence shown in SEQ ID NO. 1 in substantially purified form will generally comprise the polypeptide in a preparation in which more than 90%, e.g. 95%, 98% or 99% of the polypeptide in the preparation is that of the SEQ ID NO. 1.

A homologue of polypeptide comprising amino acid sequence shown in SEQ ID NO. 1 will be generally at least 70%, preferably at least 80 or 90% and more preferably at least 95% homologous to the polypeptide comprising the said amino acid sequence over a region of at least 20, preferably at least 30, for instance 40, 60 or 100 more contiguous amino acids. Such a polypeptide homologue will be referred to a polypeptide of the present invention.

Generally, a fragment of polypeptide comprising amino acid sequence shown in SEQ ID NO. 1 or its homologues will be at least 10, preferably at least 15, for example, 20, 25, 30, 40, 50 or 60 amino acids in length.

A cDNA capable of selectively hybridizing to the cDNA comprising nucleotide sequences shown in SEQ ID NOS. 2 or 3 will be generally at least 70%, preferably at least 80 or 90% and more preferably at least 95% homologous to the cDNA comprising the said nucleotide sequence over a region of at least 20, preferably at least 30, for instance 40, 60 or 100 or more contiguous nucleotides. Such a cDNA will be referred to "a cDNA of the present invention".

Fragments of the cDNA comprising nucleotide sequences shown in SEQ ID NOS. 2 or 3 will be at least 10, preferably at least 15, for example, 20, 25, 30 or 40 nucleotides in length, and will be also referred to "a cDNA of the present invention" as used herein.

A further embodiment of the present invention provides replication and expression vectors carrying cDNA of the present invention. The vectors may be, for example, plasmid, virus or phage vectors provided with an origin of replication, optionally a promoter for the expression of the said cDNA and optionally a regulator of the promoter. The vector may contain one or more selectable marker genes, for example, an ampicillin resistance gene. The vector maybe used in vitro, for example, of the production of RNA corresponding to the cDNA, or used to transfect a host cell.

A further embodiment of the present invention provides host cells transformed with the vectors for the replication and expression of the cDNA of the present invention, including the cDNA comprising nucleotide sequences shown in SEQ ID NOS. 2 or 3 or the open reading frame thereof. The cells will be chosen to be compatible with the vector and may for example, be bacterial, yeast, insect cells or mammalian cells.

A further embodiment of the present invention provides a method of producing a polypeptide which comprises culturing host cells of the present invention under conditions effective to express a polypeptide of the present invention. Preferably, in addition, such a method is carried out under conditions in which the polypeptide of the present invention is expressed and then produced from the host cells.

cDNA of the present invention may also be inserted into the vectors described above in an antisense orientation in order to prove for the production of antisense RNA. Such antisense RNA may be used in a method of controlling the levels of a polypeptide of the present invention in a cell.

The invention also provides monoclonal or polyclonal antibodies against a polypeptide of the present invention. The invention further provides a process for the production of monoclonal or polyclonal antibodies to the polypeptides of the present invention. Monoclonal antibodies may be prepared by common hybridoma technology using polypeptides of the present invention or fragments thereof, as an immunogen. Polyclonal antibodies may also be prepared by common means which comprise inoculating host animals, (for example, a rat or a rabbit etc.), with polypeptides of the present invention and recovering immune serum.

The present invention also provides pharmaceutical compositions containing a polypeptide of the present invention, or an antibody thereof, in association with a pharmaceutically acceptable diluent and/or carrier.

The polypeptide of the present invention specified in (1) includes that which a part of their amino acid sequence is lacking (e.g., a polypeptide comprised of the only essential sequence for revealing a biological activity in an amino acid sequence shown in SEQ ID NO. 1), that which a part of their amino acid sequence is replaced by other amino acids (e.g., those replaced by an amino acid having a similar property) and that which other amino acids are added or inserted into a part of their amino acid sequence, as well as those comprising the amino acid sequence shown in SEQ ID NO. 1.

As known well, there are one to six kinds of codon as that encoding one amino acid (for example, one kind of codon for Methionine (Met), and six kinds of codon for Leucine (Leu) are known). Accordingly, the nucleotide sequence of cDNA can be changed in order to encode the polypeptide having the same amino acid sequence.

The cDNA of the present invention, specified in (2) includes a group of every nucleotide sequence encoding polypeptides (1) shown in SEQ ID NO. 1. There is a probability that yield of a polypeptide is improved by changing a nucleotide sequence.

The cDNA specified in (3) is the embodiment of the cDNA shown in (2), and indicate the sequence of natural form.

The cDNA shown in (4) indicates the sequence of the cDNA specified in (3) with natural non-translational region.

cDNA carrying nucleotide sequence shown in SEQ ID NO. 3 is prepared by the following method:

Brief description of Yeast SST method (see USP No. 5,536,637) is as follows.

Yeast such as Saccharomyces cerevisiae should secrete invertase into the medium in order to take sucrose or raffinose as a source of energy or carbon. (Invertase is an enzyme to cleave raffinose into sucrose and melibiose, sucrose into fructose and glucose). It is known that many known mammalian signal sequence make yeast secrete its invertase. From these knowledge, SST method was developed as a screening method to find novel signal peptide which make it possible can to secrete yeast invertase from mammalian cDNA library. SST method uses yeast growth on raffinose medium as a marker. Non-secretory type invertase gene SUC2 (GENBANK Accession No. V 01311) lacking initiation codon ATG was inserted to yeast expression vector to prepare yeast SST vector pSUC2. In this expression vector, ADH promoter, ADH terminator (both were derived from AAH5 plasmid (Gammerer, Methods in Enzymol. 101, 192-201, 1983)), 2µ ori (as a yeast replication origin), TRP1 (as a yeast selective marker), ColEl ori (as a E. Coli replication origin) and ampicillin resistance gene (as a drug resistance marker) were inserted. Mammalian cDNA was inserted into the upstream of SUC2 gene to prepare yeast SST cDNA library. Yeast lacking secretory type invertase, was transformed with this library. If inserted mammalian cDNA encodes a signal peptide, yeast could survive in raffinose medium as a result of restoring secretion of invertase. Only to culture yeast colonies, prepare plasmids and determine the nucleotide sequence of the insert cDNAs, it is possible to identify novel signal peptide rapidly and easily.

Preparation of yeast SST cDNA library is as follows:
(1) mRNA is isolated from the targeted cells, double-strand synthesis is performed by using random primer with certain restriction enzyme (enzyme I) recognition site,
(2) obtained double-strand cDNA is ligated to adapter containing certain restriction endonuclease (enzyme II) recognition site, differ from enzyme I, digested with enzyme I and fractionated in a appropriate size,
(3) obtained cDNA fragment is inserted into yeast expression vector on the upstream region of invertase gene which signal peptide is deleted and the library was transformed.

Detailed description of each step is as follows:
In step (1), mRNA is isolated from mammalian organs and cell lines stimulate them with appropriate stimulator if necessary) by known methods (Molecular Cloning (Sambrook, J., Fritsch, E. F. and Maniatis, T., Cold Spring Harbor Laboratory Press, 1989) or Current Protocol in Molecular Biology (F. M. Ausubel et al, John Wiley & Sons, Inc) if not remark especially).

HUV-EC-C (endothelial cell line of vein derived from human umbilical cord: ATCC No. CRL-1730) is chosen as a cell line. Double-strand cDNA synthesis using random primer is performed by known methods.

Any sites may be used as restriction endonuclease recognition site I which is linked to adapter and restriction endonuclease recognition site II which is used in step (2), if both sites are different each other. Preferably, XhoI is used as enzyme I and EcoRI as enzyme II.

In step (2), cDNA is created blunt-ends with T4 DNA polymerase, ligated enzyme II adapter and digested with enzyme I. Fragment cDNA is analyzed with agarose-gel electrophoresis (AGE) and is selected cDNA fraction ranging in size from 300 to 800 bp. As mentioned above, any enzyme may be used as enzyme II, if it is not same the enzyme I.

In step (3), cDNA fragment obtained in step (2) is inserted into yeast expression vector on the upstream region of invertase gene which signal peptide is deleted. E. Coli was transformed with the expression vector. Many vectors are known as yeast expression plasmid vector. For example, YEp24 is also functioned in E. Coli. Preferably, pSUC2 as described above is used.

Many host E. Coli strains are known for transformation, preferably DH10B competent cell is used. Any known transformation method is available, preferably it is performed by electropolation method. Transformant is cultured by conventional methods to obtain cDNA library for yeast SST method.

However not every all of the clones do not contain cDNA fragment. Further all of the gene fragments do not encode unknown signal peptides. It is therefore necessary to screen a gene fragment encoding for an unknown signal peptide from the library.

That is to say, screening of fragments containing a sequence encoding an appropriate signal peptide is performed by transformation of the cDNA library into Saccharomyces cerevisiae (e. g. YT455 strain) which lack invertase (it may be prepared by known methods). Transformation of yeast is performed by known methods, e. g. lithium acetate method. Transformant is cultured in a selective medium, then transferred to a medium containing raffinose as a carbon source. Survival colonies are selected and then prepared plasmid. Survival colonies on a raffinose-medium indicates that some signal peptide of secretory protein was inserted to this clone.

As for isolated positive clones, the nucleotide sequence is determined. As to a cDNA encodes unknown protein, full-length clone may be isolated by using cDNA fragment as a probe and then determined to obtain full-length nucleotide sequence. These manipulation is performed by known methods.

Once the nucleotide sequence shown in SEQ ID NO. 2 is determined partially or preferably fully, it is possible to obtain cDNA encode mammalian protein itself, homologue or subset. cDNA library or mRNA derived from mammals was screened by PCR with any synthesized oligonucleotide primers or by hybridization with any fragment as a probe. It is possible to obtain cDNA encodes other mammalian homologue protein from other mammalian cDNA or genome library.

If a cDNA obtained above contains a nucleotide sequence of cDNA fragment obtained by SST (or consensus sequence thereof), it will be thought that the cDNA encodes signal peptide. So it is clear that the cDNA will be full-length or almost full (All signal peptides exist at N-termini of a protein and are encoded at 5' -temini of open reading frame of cDNA).

The confirmation may be carried out by Northern analysis with the said cDNA as a probe. It is thought that the cDNA is almost complete length, if length of the cDNA is almost the same length of the mRNA obtained in the hybridizing band.

Once the nucleotide sequence shown in SEQ ID NO. 2 is determined, cDNAs of the invention are obtained by chemical synthesis, or by hybridization making use of nucleotide fragments which are chemically synthesized as a probe. Furthermore, cDNAs of the invention are obtained in desired amount by transforming a vector that contains the cDNA into a proper host, and culturing the transformant.

The polypeptides of the present invention may be prepared by:
(1) isolating and purifying from an organism or a cultured cell,
(2) chemically synthesizing, or
(3) using recombinant DNA technology,
preferably, by the method described in (3) in an industrial production.

Examples of expression system (host-vector system) for producing a polypeptide by using recombinant DNA technology are the expression systems of bacteria, yeast, insect cells and mammalian cells.

In the expression of the polypeptide, for example, in E. Coli, the expression vector is prepared by adding the initiation codon (ATG) to 5' end of a cDNA encoding mature peptide, connecting the cDNA thus obtained to the downstream of a proper promoter (e. g., trp promoter, lac promoter, λPL promoter, T7 promoter etc.), and then inserting it into a vector (e. g., pBR322, pUC18, pUC19 etc.) which functions in an E. Coli strain.

Then, an E. Coli strain (e. g., E. Coli DH1 strain, E. Coli JM109 strain, E. Coli HB101 strain, etc.) which is transformed with the expression vector described above may be cultured in a appropriate medium to obtain the desired polypeptide. When a signal sequence of bacteria (e. g., signal sequence of pel B) is utilized, the desired polypeptide may be also released in periplasm. Furthermore, a fusion protein with other polypeptide may be also produced readily.

In the expression of the polypeptide, for example, in a mammalian cells, for example, the expression vector is prepared by inserting the cDNA encoding nucleotide sequence shown in SEQ ID NO. 3 into the downstream of a proper promoter (e. g., SV40 promoter, LTR promoter, metallothionein promoter etc.) in a proper vector (e. g., retrovirus vector, papilloma virus vector, vaccinia virus vector, SV40 vector, etc.). A proper mammalian cell (e. g., monkey COS-7 cell, Chinese hamster CHO cell, mouse L cell etc.) is transformed with the expression vector thus obtained, and then the transformant is cultured in a proper medium to express the aimed secretory protein and membrane protein of the present invention by the following method.

In case of secretory protein as for the present invention, the aimed polypeptide was expressed in the supernatant of the cells. In addition, fusion protein may be prepared by conjugating cDNA fragment encoding the other polypeptide, for example, Fc portion of antibody.

On the other hand, in case of membrane protein as for the present invention, the aimed polypeptide was expressed on the cell membrane. A cDNA encoding the nucleotide sequence of SEQ ID NO. 2 with deletion of extracellular region was inserted into the said vector, transfected into the an adequate mammalian cells to secret the aimed soluble polypeptide in the culture medium. In addition, fusion protein may be prepared by conjugating cDNA fragment encoding the said mutant with deletion of extracellular region and other polypeptide, for example, Fc portion of antibody.

The polypeptide available by the way described above can be isolated and purified by conventional biochemical method.

### Industrial Applicability

It is considered that the polypeptide of the present invention and a cDNA which encodes the polypeptide will show one or more of the effects or biological activities (including those which relates to the assays cited below). The effects or biological activities described in relation to the polypeptide of the present invention are provided by administration or use of the polypeptide or by administration or use of a cDNA molecule which encodes the polypeptide (e.g., vector suitable for gene therapy or cDNA introduction).

### [Cytokine activity and cell proliferation/differentiation activity]

The protein of the present invention may exhibit cytokine, cell proliferation (either inducing or inhibiting) or cell differentiation (either inducing or inhibiting) activity or may induce production of other cytokines in certain cell populations. Many protein factors discovered to date, including all known cytokines, have exhibited activity in one or more factor dependent cell proliferation assays, and hence the assays serve as a convenient confirmation of cytokine activity. The activity of a polypeptide of the present invention is evidenced by any one of a number of routine factor dependent cell proliferation assays for cell lines.

### [Immune stimulating/suppressing activity]

The protein of the present invention may also exhibit immune stimulating or immune suppressing activity. The protein of the present invention may be useful in the treatment of various immune deficiencies and disorders (including severe combined immunodeficiency (SCID)), e.g., in regulating (up or down) growth and proliferation of T and/or B lymphocytes, as well as effecting the cytolytic activity of NK cells and other cell populations. These immune deficiencies may be genetic or be caused by viral infection such as AIDS (HIV) as well as bacterial or fungal infections, or may result from autoimmune disorders. More specifically, infectious diseases causes by viral, bacterial, fungal or other infection may be treatable using the polypeptide of the present invention, including AIDS (HIV), infections by hepatitis viruses, herpes viruses, mycobacteria, leshmania, malaria and various fungal infections such as candida. Of course, in this regard, the protein of the present invention may also be useful where a boost to the immune system generally would be indicated, i.e., in the treatment of cancer.

The protein of the present invention may be useful in the treatment of allergic reactions and conditions, such as asthma or other respiratory problems. The protein of the present invention may also be useful in the treatment of the other conditions required to suppress the immuno system (for example, asthma or respiratory disease.)

The protein of the present invention may also suppress chronic or acute inflammation, such as, for example, that associated with infection such as septic shock or inflammatory bowel disease such as systemic inflammatory response syndrome (SIRS), Crohn's disease or resulting from over production of cytokines such as TNF or IL-I wherein the effect was demonstrated by IL- 11.

### [Hematopoiesis regulating activity]

The protein of the present invention may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis. The said biological activities are concerned with the following all or some example(s). e.g. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (i.e., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either in-vitro or ex-vivo (i.e. in conjunction with bone marrow transplantation) as normal cells or genetically manipulated for gene therapy.

The activity of the protein of the present invention may, among other means, be measured by the following methods :

### [Tissue generation/regeneration activity]

The protein of the present invention also may have utility in compositions used for bone, cartilage, tendon, ligament and/or nerve tissue growth or regeneration, as well as for wound healing and tissue repair, and in the treatment of bums, incisions and ulcers.

The protein of the present invention, which induces cartilage and/or bone growth in circumstances where bone is not normally formed, may be applied to the healing of bone fractures and cartilage damage or defects in humans and other animals. Such a preparation employing the protein of the present invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery.

The protein of the present invention may also be used in the treatment of periodontal disease, and in other tooth repair processes. Such agents may provide an environment to attract bone-forming cells, stimulate growth of bone-forming cells or induce differentiation of progenitors of bone-forming cells. The protein of the present invention may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to the protein of the present invention is tendon/ligament formation. The protein of the present invention, which induces tendon/ligament-like tissue or other tissue formation in circumstances where such tissue is not normally formed, may be applied to the healing of tendon or ligament tears, deformities and other tendon or ligament defects in humans and other animals. Such a preparation employing the protein inducing a tendon/ligament-like tissue may have prophylactic use in preventing damage to tendon or ligament tissue, as well as use in the improved fixation of tendon or ligament to bone or other tissues, and in repairing defects to tendon or ligament tissue. De novo tendon/ligament-like tissue formation induced by a composition of the present invention contributes to the repair of congenital, trauma induced, or other tendon or ligament defects of other origin, and is also useful in cosmetic plastic surgery for attachment or repair of tendons or ligaments. The compositions of the present invention may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors ex vivo for return in vivo to effect tissue repair. The compositions of the present invention may also be useful in the treatment of tendinitis, Carpal tunnel syndrome and other tendon or ligament defects. The compositions may also include an appropriate matrix and/or sequestering agent as a carrier as is well known in the art.

The protein of the present invention may also be useful for proliferation of neural cells and for regeneration of nerve and brain tissue. i.e. for the treatment of central and peripheral nervous system diseases and neuropathies. as well as mechanical and traumatic disorders, which involve degeneration, death or trauma to neural cells or nerve tissue. More specifically, the protein of the present invention may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the invention include mechanical and traumatic disorders, such as spinal cord disorders, head trauma and cerebrovascular diseases such as stroke. Peripheral neuropathies resulting from chemotherapy or other medical therapies may also be treatable using the polypeptide of the present invention.

It is expected that the protein of the present invention may also exhibit activity for generation of other tissues, such as organs (including, for example, pancreas, liver, intestine, kidney, skin, endothelium), muscle (smooth, skeletal or cardiac) and vascular (including vascular endothelium) tissue, or for promoting the proliferation of cells comprising such tissues. Part of the desired effects may be by inhibition of fibrotic scarring to allow normal tissue to regenerate.

The protein of the present invention may also be useful for gut protection or regeneration and treatment of lung or liver fibrosis, reperfusion injury in various tissues, and conditions resulting from systemic cytokine damage.

### [Activin/Inhibin activity]

The protein of the present invention may also exhibit activin- or inhibin-related activities. Inhibins are characterized by their ability to inhibit the release of follicle stimulating hormone (FSH), while activins are characterized by their ability to stimulate the release of follicle stimulating hormone (FSH). Thus, the protein of the present invention alone or in heterodimers with a member of the inhibin ^{*}a family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein of the present invention, as a homodimer or as a heterodimer with other protein subunits of the inhibin-*b group, may be useful as a fertility inducing therapeutic, based upon the ability of activin molecules in stimulating FSH release from cells of the anterior pituitary (See USP 4,798,885). The protein of the present invention may also be useful for advancement of the onset of fertility in sexually immature mammals, so as to increase the lifetime reproductive performance of domestic animals such as cows, sheep and pigs.

### [Chemotactic/chemokinetic activity]

The protein of the present invention may have chemotactic or chemokinetic activity e.g., functioning as a chemokine, for mammalian cells, including, for example, monocytes, neutrophils, T-cells, mast cells, eosinophils and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

If a protein or peptide can stimulate, directly or indirectly, the directed orientation or movement of such cell population, it has chemotactic activity for a particular cell population. Preferably, the protein or peptide has the ability to directly stimulate directed movement of cells. Whether a particular protein has chemotactic activity for a population of cells can be readily determined by employing such protein or peptide in any known assay for cell chemotaxis.

### [Hemostatic and thrombolytic activity]

The protein of the present invention may also exhibit hemostatic or thrombolyic activity. As a result, such a protein is expected to be useful in treatment of various coagulation disorders (including hereditary disorders, such as hemophilias) or to enhance coagulation and other hemostatic events in treating wounds resulting from trauma, surgery or other causes. A protein of the present invention may also be useful for dissolving or inhibiting formation of thromboses and for treatment and prevention of conditions resulting therefrom such as, for example, infarction or stroke.

### [Receptor/ligand activity]

The protein of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including cellular adhesion molecules such as Selectins, Integrins and their ligands) and receptor/ligand pairs involved in antigen presentation, antigen recognition and development of cellular and humoral immune responses. Receptors and ligands are also useful for screening of potential peptide or small molecule inhibitors of the relevant receptor/ligand interaction. The protein of the present invention (including, without limitation, fragments of receptors and ligands) may themselves be useful as inhibitors of receptor/ligand interactions.

### [Other activity]

The protein of the present invention may also exhibit one or more of the following additional activities or effects: inhibiting growth of or killing the infecting agents including bacteria, viruses, fungi and other parasites; effecting (suppressing or enhancing) body characteristics including height, weight, hair color, eye color, skin, other tissue pigmentation, or organ or body part size (for example, breast augmentation or diminution) etc.; effecting elimination of dietary fat, protein, carbohydrate; effecting behavioral characteristics including appetite, libido, stress, cognition (including cognitive disorders), depression and violent behaviors; providing analgesic effects or other pain reducing effects; promoting differentiation and growth of embryonic stem cells in lineages other than hematopoietic lineages; in the case of enzymes, correcting deficiencies of the enzyme and treating deficiency-related diseases.

The protein with above activities, is suspected to have following functions by itself or interaction with its ligands or receptors or association with other molecules. For example, proliferation or cell death of B cells, T cells and/or mast cells; specific induction by promotion of class switch of immunoglobulin genes; differentiation of B cells to antibody-forming cells; proliferation, differentiation, or cell death of precursors of granulocytes; proliferation, differentiation, or cell death of precursors of monocytes-macrophages; proliferation, of up regulation or cell death of neutrophils, monocytes-macrophages, eosinophils and/or basophils; proliferation, or cell death of precursors of megakaryocytes; proliferation, differentiation, or cell death of precursors of neutrophils; proliferation, differentiation, or cell death of precursors of T cells and B cells; promotion of production of erythrocytes; sustainment of proliferation of erythrocytes, neutrophils, eosinophils, basophils, monocytes-macrophages, mast cells, precursors of megakaryocyte; promotion of migration of neutrophils, monocytes-macrophages, B cells and/or T cells; proliferation or cell death of thymocytes; suppression of differentiation of adipocytes; proliferation or cell death of natural killer cells; proliferation or cell death of hematopoietic stem cells; suppression of proliferation of stem cells and each hematopoietic precursor cells; promotion of differentiation from mesenchymal stem cells to osteoblasts or chondrocytes, proliferation or cell death of mesenchymal stem cells, osteoblasts or chondrocytes and promotion of bone absorption by activation of osteoclasts and promotion of differentiation from monocytes to osteoclasts.

The polypeptide of the present invention is also suspected to function to nervous system, so expected to have functions below; differentiation to kinds of neurotransmitter-responsive neurons, survival or cell death of these cells; promotion of proliferation or cell death of glial cells; spread of neural dendrites; survival or cell death of gangriocytes; proliferation, promotion of differentiation, or cell death of astrocytes; proliferation, survival or cell death of peripheral neurons; proliferation or cell death of Schwann cells; proliferation, survival or cell death of motoneurons.

Furthermore, in the process of development of early embryonic, the polypeptide of the present invention is expected to promote or inhibit the organogenesis of epidermis, brain, backbone, and nervous system by induction of ectoderm, that of notochord connective tissues (bone, muscle, tendon), hemocytes, heart, kidney, and genital organs by induction of mesoderm, and that of digestive apparatus (stomach, intestine, liver, pancreas), respiratory apparatus (lung, trachea) by induction of endoderm. In adult, also, this polypeptide is thought to proliferate or inhibit the above organs.

Therefore, the polypeptide of the present invention itself is expected to be used as an agent for the prevention or treatment of disease of progression or suppression of immune, nervous, or bone metabolic function, hypoplasia or overgrowth of hematopoietic cells: for example, inflammatory disease (rheumatism, ulcerative colitis, etc.), decrease of hematopoietic stem cells after bone marrow transplantation, decrease of leukocytes, platelets, B-cells, or T-cells after radiation exposure or chemotherapeutic dosage against cancer or leukemia, anemia, infectious disease, cancer, leukemia, AIDS, bone metabolic disease (osteoporosis etc.), various degenerative disease (Alzheimer's disease, multiple sclerosis, etc.), or nervous lesion.

In addition, since the polypeptide of the present invention is thought to induce the differentiation or growth of organs derived from ectoderm, mesoderm, and endoderm, this polypeptide is expected to be an agent for tissue repair (epidermis, bone, muscle, tendon, heart, kidney, stomach, intestine, liver, pancreas, lung, and trachea, etc.).

By using polyclonal or monoclonal antibodies against the polypeptide of the present invention, quantitation of the said polypeptide in the body can be performed. It can be used in the study of relationship between this polypeptide and disease or diagnosis of disease, and so on. Polyclonal and monoclonal antibodies can be prepared using this polypeptide or its fragment as an antigen by conventional methods.

Identification, purification or molecular cloning of known or unknown proteins which bind the polypeptide of the present invention (preferably polypeptide of extracellular domain) can be performed using the polypeptide of the present invention by, for example, preparation of the affinity-column.

Identification of the downstream signal transmission molecules which interact with the polypeptide of the present invention in cytoplasma and molecular cloning of the gene can be performed:
by west-western method using the polypeptide of the present invention (preferably polypeptide of transmembrane region or intracellular domain) or
by yeast two-hybrid system using the cDNA (preferably cDNA encoding transmembrane region or cytoplasmic domain of the polypeptide).

Agonists/antagonists of this receptor polypeptide and inhibitors between receptor and signal transduction molecules can be screened using the polypeptide of the present invention.

cDNAs of the present invention are useful not only the important and essential template for the production of the polypeptide of the present invention which is expected to be largely useful, but also be useful for diagnosis or therapy (for example, treatment of gene lacking, treatment to stop the expression of the polypeptide by antisense DNA (RNA)). Genomic DNA may be isolated with the cDNA of the present invention, as a probe. As the same manner, a human gene encoding which can be highly homologous to the cDNA of the present invention, that is, which encodes a polypeptide highly homologous to the polypeptide of the present invention and a gene of animals excluding mouse which can be highly homologous to the cDNA of the present invention, also may be isolated.

### [Application to Medicaments]

The polypeptide of the present invention or the antibody specific for the polypeptide of the present invention is administered systemically or topically and in general orally or parenterally, preferably parenterally, intravenously and intraventricularly, for preventing or treating the said diseases.

The doses to be administered depend upon age, body weight, symptom, desired therapeutic effect, route of administration, and duration of the treatment etc. In human adults, one dose per person is generally between 100 µg and 100 mg, by oral administration, up to several times per day, and between 10 µg and 100 mg, by parental administration up to several times per day.

As mentioned above, the doses to be used depend upon various conditions. Therefore, there are cases in which doses lower than or greater than the ranges specified above may be used.

The compounds of the present invention, may be administered as solid compositions, liquid compositions or other compositions for oral administration, as injections, liniments or suppositories etc. for parental administration.

Solid compositions for oral administration include compressed tablets, pills, capsules, dispersible powders, and granules. Capsules include soft or hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric materials (such as sugar, gelatin, hydroxypropyl cellulose or hydroxypropylmethyl cellulose phthalate, etc.), or be coated with more than two films. And then, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used (purified water, ethanol etc.). Besides inert diluents, such compositions may also comprise adjuvants (such as wetting agents, suspending agents, etc.), sweetening agents, flavoring agents, perfuming agents, and preserving agents.

Other compositions for oral administration include spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parental administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s)(propylene glycol, polyethylene glycol, olive oil, ethanol, POLYSOLBATE 80 (Trade mark) etc.).

Injections may comprise additional compound other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

### Best Mode carrying out the Invention

The invention is illustrated by the following examples relate to clone 0AH047, but not limit the invention.

### Example 1: Preparation of poly(A)⁺RNA

Total RNA was extracted from endothelial cell line of vein derived from human umbilical cord (HUV-EC-C) by using TRIzol Reagent (Trade mark, marketed by GIBCOBRL Co.). Poly(A)⁺RNA was purified by mRNA Purification Kit (Trade mark, marketed by Pharmacia).

### Example 2: Preparation of yeast SST cDNA library

Double strand cDNA was synthesized by Super Script Plasmid System for cDNA Synthesis and Plasmid Cloning (Trade name, marketed by GIBCOBRL Co.) with above poly(A)⁺RNA as template and random 9mer as primer which was containing XhoI site: 5'-CGATTGAATTCTAGACCTGCCTCGAGNNNNNNNNN-3' (SEQ ID NO. 4). cDNA was ligated EcoRI adapter (marketed by GIBCOBRL Co.) by DNA ligation kit ver. 2 (Trade name, marketed by Takara-Shuzo Co., this kit was used in all ligating steps hereafter) and digested by XhoI. cDNAs were separated by agarose-gel electrophoresis. 300 - 800 bp cDNAs were isolated and were ligated to EcoRI/NotI site of pSUC2 (see US Patent No. 5,536,637). E. Coli DH10B strains were transformed by pSUC2 with electropolation to obtain yeast SST cDNA library.

### Example 3: Screening by SST method and determination of nucleotide sequence of SST positive clone

Plasmids of the said cDNA library were prepared. Yeast YTK12 strains were transformed by the plasmids with lithium acetate method (Current Protocols In Molecular Biology 13.7.1). The transformed yeast were plated on triptphan-free medium (CMD-Trp medium) for selection. The plate was incubated for 48 hour at 30°C. Replica of the colony (transformant) which was obtained by Accutran Replica Plater (Trade name, marketed by Schleicher & Schuell Co.) were placed onto YPR plate containing raffinose for carbon source, and the plate was incubated for 14 days at 30°C. After 3 days, each colony appeared was streaked on YPR plate again. The plates were incubated for 48 hours at 30°C. Single colony was inoculated to YPD medium and was incubated for 48 hours at 30°C. Then plasmids were prepared. Insert cDNA was amplified by PCR with two kind primers which exist end side of cloning site on pSUC2 (sense strand primers were biotinylated). Biotinylated single strand of cDNAs were purified with Dynabeads (Trade name, marketed by DYNAL Co.) and the nucleotide sequences were determined. Sequencing was performed by Dye Terminator Cycle Sequencing Ready Reaction with DNA Sequencing kit (Trade name, marketed by Applied Biosystems Inc.) and sequence was determined by DNA sequencer 373 (Applied Biosystems Inc.) (All sequencing hereafter was carried out with this method).

We tried to carry out cloning of full-length cDNA which was proved to be new one according to the homology search for the obtained nucleotide sequences and deduced amino acid sequences in data base.

### Example 4: Cloning of a full-length cDNA and determination of nucleotide sequence

A full-length cDNA was cloned using Marathon cDNA Amplification Kit (Trade name, marketed by Clontech Co.) according to 3'RACE (Rapid Amplification of cDNA End) method. Double strands cDNA was prepared from poly(A)⁺RNA in each clone, i.e., endothelial cell line of vein derived from human umbilical cord. 27mer primer OAH047-F1: 5'-GCGACACGTGGATCCAAGATGGCGACG-3' (SEQ ID NO. 5) containing the deduced initiation ATG codon region based on the information of nucleotide sequence obtained by SST, was prepared. PCR was performed with the said primer and adapter primer attached in the kit. A cDNA which was amplified with clone 0AH047 specifically, was separated with agarose-gel electrophoresis, ligated to pT7 Blue-2 T-Vector (Trade name, marketed by Novagen Co.) and transfected into E. Coli DH5a to prepare the plasmid. Nucleotide sequences of 5'-end were determined, and the existence of nucleotide sequence 0AH047 SST cDNA was confirmed. Nucleotide sequence of full-length 0AH047 SST cDNA was determined and then sequence shown in SEQ ID N0. 3 was obtained. An open reading frame was determined and deduced amino acid sequence and nucleotide sequence shown in SEQ ID NOS. 1 and 2, respectively, were obtained.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence and the nucleotide sequence of 0AH047 of the present invention. In addition, the polypeptide of the present invention was expected to possess the transmembrane region by hydrophobisity analysis of the obtained amino acid sequence. From these results, it was proved that polypeptide of the present invention was new membrane protein. Further, the search using BLASTX, BLASTP and FASTA revealed a significant homology between clone 0AH047 (region of 12th - 642nd amino acid in SEQ ID NO. 1) and yeast endomembrane protein (Genbank Accession U53880, region of 11th - 667th amino acid). Based on these homologies, clone OAH047 was expected to functionate transportation of hormone or the corresponding molecular from cell membrane to lysosome as a transpotor.

## Claims

1. A substantially purified form of a polypeptide that comprising the amino acid sequence shown in SEQ ID NO. 1, a homologue thereof, a fragment thereof or a homologue of the fragment.

2. A polypeptide according to claim 1 that comprising the amino acid sequence shown in SEQ ID NO. 1.

3. A cDNA encoding the polypeptide according to claim 1.

4. A cDNA according to claim 3 that comprising the nucleotide sequence shown in SEQ ID NO. 2, or a fragment cDNA selectively hybridized to the cDNA.

5. A cDNA according to claim 3 that comprising the nucleotide sequence shown in SEQ ID NO. 3, or a fragment cDNA selectively hybridized to the cDNA.

6. A replication or expression vector carrying the cDNA according to claims 3 to 5.

7. A host cell transformed with the replication or expression vector according to claim 6.

8. A method for producing the polypeptide according to claim 1 or 2 which comprises culturing a host cell according to claim 7 under a condition effective to express the polypeptide according to claim 1 or 2.

9. A monoclonal or polyclonal antibody against the polypeptide according to claim 1 or 2.

10. A pharmaceutical composition containing the polypeptide according to claim 1 or 2 or the antibody according to claim 9, in association with pharmaceutically acceptable diluent and/or carrier.
